(19) Europäisches Patentamt / European Patent Office / Office européen des brevets

(11) **EP 2 468 244 A1**

(12) **EUROPEAN PATENT APPLICATION**
published in accordance with Art. 153(4) EPC

(43) Date of publication:
   **27.06.2012 Bulletin 2012/26**

(21) Application number: **10810021.5**

(22) Date of filing: **20.08.2010**

(51) Int Cl.:
   *A61K 8/34* (2006.01)   *A61K 8/02* (2006.01)
   *A61K 8/60* (2006.01)   *A61K 8/72* (2006.01)
   *A61Q 5/06* (2006.01)

(86) International application number:
   **PCT/JP2010/064048**

(87) International publication number:
   **WO 2011/021680 (24.02.2011 Gazette 2011/08)**

(84) Designated Contracting States:
   **AL AT BE BG CH CY CZ DE DK EE ES FI FR GB
   GR HR HU IE IS IT LI LT LU LV MC MK MT NL NO
   PL PT RO SE SI SK SM TR**

(30) Priority: **20.08.2009 JP 2009191485**

(71) Applicant: **Shiseido Company, Ltd.
   Chuo-ku
   Tokyo 104-8010 (JP)**

(72) Inventors:
   • **TOYODA, Tomonori
     Yokohama-shi
     Kanagawa 224-8558 (JP)**

   • **FUJIYAMA, Taizo
     Yokohama-shi
     Kanagawa 224-8558 (JP)**
   • **KURASHIMA, Takumi
     Yokohama-shi
     Kanagawa 224-8558 (JP)**

(74) Representative: **Henkel, Breuer & Partner
   Patentanwälte
   Maximiliansplatz 21
   80333 München (DE)**

(54) **HAIR-DRESSING COSMETIC**

(57)    Disclosed is a hair-styling cosmetic which exerts good hair-styling effect and hair-restyling effect, although being water-based and having a low viscosity, shows no stickiness and high smoothness and gives the sense of a favorable light finish. Specifically disclosed is a hair-styling cosmetic containing 3-30% by mass of one or more kinds of components selected from among a sugar alcohol, a polyalkylene glycol and derivatives thereof, **characterized by** having such an adhesiveness as showing a ball number of 1-30 in the inclined ball tack test (inclination angle 10˚, measured at 25˚C, humidity 50%), and a viscosity of 100 mPa·s or less (measured with a B-type viscometer at 25˚C) . Thus, a hair-styling cosmetic, which exerts good hair-styling effect and hair-restyling effect, shows no stickiness and high smoothness, gives the sense of a favorable light finish, and is suitable for application by mist-spraying, can be obtained.

[Figure 1]

**Description**

TECHNICAL FIELD

The present invention relates to a hair-styling cosmetic. More specifically, the present invention relates to a hair-styling cosmetic that is excellent in hair-styling effect and hair-restyling effect although having a low viscosity, shows no stickiness, shows smoothness, and gives excellent light finish.

BACKGROUND ART

Conventionally, hair setting resins such as a hair fixing polymer and a coating-formable polymer are incorporated for styling in hair-styling cosmetics. However, hair setting resins have defects that they provide stiffness, unevenness of a coating, decrease in styling keeping effect under a high humidity, and the like. Therefore, in order to eliminate the defects, various countermeasures have been adopted.

For example, JP-A-2007-217314 (Patent Document 1) describes that a spray-form powder cosmetic comprising a hair fixing polymer compound, a polyhydric alcohol, a monohydric alcohol and a propellant by respective specific amounts is excellent in hair-restyling property, gives no sticky feeling and has natural glossy feeling.

JP-A-H11-100312 (Patent Document 2) describes that a hair cosmetic comprising a specific low viscosity polyether compound and a specific polymer resin compound by respective specific amounts has hair-styling effect and hair-styling keeping effect, gives no stickiness and stiffness, and enables hair-restyling by passing the fingers through hair even after drying.

JP-A-H3-261713 (Patent Document 3) describes that a hair cosmetic comprising a specific polyoxyalkylene-based compound and/or polyoxyalkylenealkyl glycoside, a specific hair fixing polymer compound and a specific high molecular weight polyethylene glycol (molecular weight: 6,000 to 30,000) has hair-styling property and smoothing property (smoothness).

JP-A-2002-167317 (Patent Document 4) describes that a hair cosmetic composition comprising an amphoteric polymer, a sugar alcohol and a sugar alcohol derivative (for example, a polyoxyalkylene-adduct of a sugar alcohol, and the like) has hair-styling effect and set keeping effect, and gives no sticky feeling and stiff feeling.

JP-A-2004-505902 (Patent Document 5) describes that a hair-care composition comprising a specific water-soluble polyalkylene glycol and a specific coating-forming polymer in a specific ratio and further comprising a liquid carrier is excellent in re-styling property and feeling.

However, the above-mentioned publications specifically fail to describe or suggest the objects or problems to obtain hair-styling effect and hair-restyling effect that can be satisfied sufficiently in a water-based low viscosity hair-styling cosmetic, and to obtain a hair-styling cosmetic that exhibits effects that the cosmetic gives no sticky feeling, is smooth and gives excellent light finish. In a water-based hair-styling cosmetic having a low viscosity, it is especially difficult to balance setting effect and arrangement effect. Therefore, development of a water-based low viscosity hair-styling cosmetic that has sufficient setting effect and arrangement effect in combination and also has fine feeing of use has been required.

PRIOR Art DOCUMENTS

PATENT DOCUMENTS

Patent Document 1: JP-A-2007-217314

Patent Document 2: JP-A-H11-100312

Patent Document 3: JP-A-H3-261713

Patent Document 4: JP-A-2002-167317

Patent Document 5: JP-A-2004-505902

SUMMARY OF INVENTION

PROBLEM TO BE SOLVED BY THE INVENTION

The present invention has been made in view of the above-mentioned conventional situation, and aims at providing a hair-styling cosmetic that is excellent in hair-styling effect and hair-restyling effect although being an aqueous-based and having a low viscosity, shows no stickiness, shows smoothness and gives excellent light finish.

MEANS FOR SOLVING PROBLEM

Namely, the invention provides a hair-styling cosmetic comprising (a) a sugar alcohol and/or a polyalkylene glycol by from 3 to 30% by mass, **characterized in that** the cosmetic has such an adhesiveness as showing a ball number of from 1 to 30 in an inclined ball tack test (inclination angle 10˚, measured at 25˚C) and has a viscosity of 100 mPa·s or less (25˚C, a B-type viscometer).

EFFECT OF THE INVENTION

Since the hair-styling cosmetic of the present invention incorporates predetermined amounts of the specific components: one kind or two kinds or more selected from sugar alcohols, polyalkylene glycols and derivatives thereof, and has specific adhesiveness and viscosity, it is suitable for use by atomizing into a form of mist and can exhibit excellent hair-styling effect and hair-restyling effect.

BRIEF DESCRIPTION OF DRAWINGS

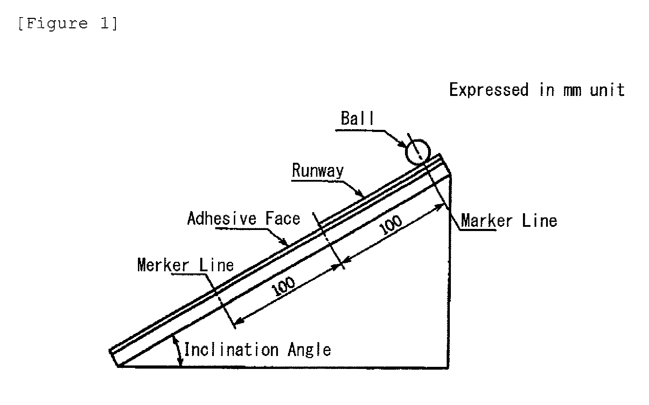

    FIG. 1 is a schematic view showing an apparatus for a ball tack test.

MODE FOR CARRYING OUT THE INVENTION

The hair-styling cosmetic of the present invention comprises one kind or two kinds or more selected from sugar alcohols, polyalkylene glycols and derivatives thereof (component a) as an essential component.
The sugar alcohol used in the present invention is a polyhydric alcohol obtained by reducing the carbonyl group of a sugar. Specifically, maltitol ("Malbit"; manufactured by B Food Science Co., Ltd.), sorbitol ("Sorbitol C" ; manufactured by B Food Science), ribitol, mannitol, arabitol, galactitol, xylitol, erythritol, inositol and the like can be exemplified. Alternatively, a derivative of a sugar alcohol can also be used in the present invention. For example, POE-POP-added (oleic acid polyoxyethylene sorbit "Rheodol 430V": Kao Corporation, polyoxypropylene sorbit "Uniol HS-1600D": NOF Corporation), alkyl group-added, cationized, anionized and silylated derivatives, and the like can be exemplified. Among these, sorbitol and maltitol are preferably used from the viewpoints of absence of stickiness and stiffness, and the like.
Preferable examples of the polyalkylene glycols and derivative thereof used in the present invention may include EO polymers in which ethylene oxide (EO) constitutional units have been polymerized, PO polymers in which propylene oxide (PO) constitutional units have been polymerized, BO polymers in which butylene oxide (BO) constitutional units have been polymerized, and copolymers in which the above-mentioned constitutional units have been copolymerized, and the like. Specifically, EO polymers, EO-PO copolymers comprising EO constitutional units and PO constitutional units, EO-BO copolymers comprising EO constitutional units and BO constitutional units, and the like are preferable. The format of copolymerization is not specifically limited, and is any of block copolymerization, graft copolymerization, random copolymerization and the like.
As the polyalkylene glycols used in the present invention, commercially available ones can be utilized, and examples can include EO-adduct polymers: PEG200, PEG300, PEG400, PEG600, PEG1000, PEG1540, PEG2000, PEG4000, PEG6000, PEG11000 and PEG20000 (NOF Corporation or Toho Chemical Industry Co., Ltd.), PO-adduct polymers: Uniol D-700, Uniol D-1000, Uniol D-1200 and Uniol D-2000 (NOF Corporation), and the like. Although the polyalkylene glycols used in the present invention are not specifically limited, among those, polyalkylene glycols are optimal.
The polyalkylene glycol derivatives used in the present invention encompass the following hair-styling oil component and the like. The hair-styling oil component means an EO/PO adduct of a mono- to tetravalent alcohol or a mono- to trivalent carboxylic acid. Commercially available ones can be utilized, and examples can include Unilub 50MB168, Unilub MB370, Triol G-40, Savondor SGP-7 and Savondor GP-9 (NOF Corporation), and the like.

The incorporation amount of the sugar alcohols, polyalkylene glycols and/or derivatives thereof (component a) in the cosmetic of the present invention is from 3 to 30% by mass, more preferably from 5 to 25% by mass. When the incorporation amount is less than 3% by mass, a desired effect may not be obtained, and when the component is incorporated by going beyond 30% by mass, stickiness and stiffness may be caused.

The hair-styling cosmetic of the present invention exhibits adhesiveness by the volatilization of the solvent in the cosmetic, and needs to have predetermined adhesiveness. In the present specification, the adhesiveness of the cosmetic is specified by a ball tack test according to JIS Z 0237.

The ball tack test is a method for testing adhesiveness comprising measuring by using a measurement apparatus as shown in FIG. 1. Specifically, when a sample to be measured (adhesive material) is disposed on the inclined plane of the measurement apparatus of FIG. 1, the part from the top of the inclined plane to the position at 10 cm from the top is covered with a non-adhesive sheet to form an entrance path, and balls each made of a predetermined material and having a predetermined size are each rolled from the top of the inclined plane, the adhesiveness is specified by the number of the ball having the maximum size among the balls that have stopped on either position of the adhesive surface.

The hair-styling cosmetic of the present invention has such an adhesiveness as showing a ball number of from 1 to 30, more preferably from 3 to 25 when a coating having a thickness of 0.1 mm by using the cosmetic and drying at 25˚C under a humidity of 50% and the coating is measured by a ball tack test according to JIS Z 0237 at an inclination angle of the inclined plane in the measurement apparatus shown in FIG. 1 of 10˚, at 25˚C and a humidity of 50%. In order to exert hair-restyling effect, adhesiveness in this range is preferable.

When the adhesiveness is so low that the ball No. 1 is not stopped, hair-restyling effect is insufficient, whereas when the adhesiveness is so high that a ball larger than the ball No. 30 is stopped, hair becomes sticky.

Furthermore, since the hair-styling cosmetic of the present invention generates adhesiveness immediately during application to hair and exhibits adhesiveness by the volatilization of the solvent in the composition, it needs to have a viscosity measured with a B-type viscometer at 25˚C of 100 mPa·s or less. Furthermore, the effect of the present invention is exerted by atomizing into a form of mist. When the viscosity exceeds 100 mPa·s, the cosmetic may become unsuitable for atomizing the cosmetic into a form of mist, for example, problems such as clogging in a nozzle of an atomizing apparatus may be caused.

It is preferable that the hair-styling cosmetic of the present invention comprises a hair setting resin in addition to the above-mentioned component (a). By incorporating the hair setting resin, humidity resistance is improved.

The hair setting resin that may be incorporated in the cosmetic of the present invention is an anionic, cationic, amphoteric or nonionic coating-forming polymer, which is also called a setting agent, a setting resin or the like, and may be one that has been conventionally incorporated in hair styling agents since before. Specific examples can include the following ones.

Acrylic-based and vinyl-based hair setting resins:

As anionic hair setting resins, alkyl acrylate-diacetoneacrylamide copolymers (Plas Cize L-53P, Plas Cize L-9909B, Plas Cize L-9948B and the like (manufactured by Goo Chemical Co., Ltd.)), alkyl acrylate-octylacrylamide copolymers (Dermacryl79 (manufactured by Nippon NSC Ltd.), polyethylene glycol-polypropylene glycol-25 dimethycone acrylates copolymers (Luviflex SILK (manufactured by BASF), acrylic acid-acrylamide-ethyl acrylate copolymers (Ultrahold 8, Ultrahold Strong (manufactured by BASF), alkyl acrylate copolymers (Aniset NF-1000, Aniset HS-3000 and the like (manufactured by Osaka Organic Chemical Industry Ltd.)), and the like.

Amphoteric hair setting resins:

Octylamide acrylate-hydroxypropylpropyl acrylate-butylaminoethyl methacrylate (AMPHOMER SH30, AMPHOMER LV-71 (manufactured by Nippon NSC Ltd.)), methacryloyloxyethylcarboxybetaine-alkyl methacrylate copolymers (Yukaformer R205, Yukaformer 301, Yukaformer SM, Yukaformer 104D and the like (manufactured by Mitsubishi Chemical Corporation), RAM Resin-1000, RAM Resin-2000, RAM Resin-3000 and RAM resin-4000 (manufactured by Osaka Organic Chemical Industry Ltd.)), dimethyldiallylammonium chloride-acrylic acid copolymers (Mercoat 280 and Mercoat 295 (manufactured by Nalco)), dimethyldiallylammonium chloride-acrylamide-acrylic acid copolymers (Mercoat Plus 3330 and Mercoat Plus 3331 (manufactured by Nalco)), and the like.

Cationic hair setting resins:

Diethyl sulphates of vinylpyrrolidone-dimethylaminoethyl methacrylate copolymers (H. C. Polymer 1S (M) and H. C. Polymer 2 (manufactured by Osaka Organic Chemical Industry Ltd.), Gafcoat 755N (manufactured by ISP), vinylpyrrolidone-dimethylaminopropylmethacrylamide-lauryldi methylaminopropylmethacrylamide copolymers (Stylese W-20 (manufactured by ISP), vinylpyrrolidone-N,N-dimethylaminoethyl methacrylate-alkyl acrylate-tripropylene glycol diacrylate copolymers (Coscat GA467, Coscat GA468 (manufactured by Osaka Organic Chemical Industry Ltd.), polydimethylmethylenepiperidinium chloride (Mercoat 100 (manufactured by Nalco), dimethyldiallylammonium chloride-acrylamide copolymers (Mercoat 550 (manufactured by Nalco)), trimethylaminopropylacrylamide chliride-dimethylacrylamide copolymers, and the like.

Nonionic hair setting resins:

Polyvinylpyrrolidone (Luviskol K17, Luviskol K30 and Luviskol K90 (manufactured by BASF), PVP K (manufactured by ISP), vinylpyrrolidone-vinyl acetate copolymers (PVP/VA S-630, PVP/VA E-735 and PVP/VA E-335 (manufactured by ISP)), Luviskol VA73W and Luviskol 37E (manufactured by BASF), PVA-6450 (manufactured by Osaka Organic Chemical Industry Ltd.), vinyl methyl ether-alkyl maleate copolymers (Gantrez A-425, Gantrez ES-225, Gantrez ES-335 and the like (manufactured by ISP)), vinylpyrrolidone-methacrylamide-vinylimidazole copolymers (Luviset Clear (manufactured by BASF)), polyvinylcaprolactam (Luviskol Plus (manufactured by BASF)), and the like.

Urethane-based hair setting resins:

Yodosol PUD (manufactured by Nippon NSC Ltd.), Luviset P. U. R. (manufactured by BASF), polymers described in Jpn. Pat. Appln. KOKAI Publication No. 2006-213706, and the like; as acrylic-urethane-based polymers, Dynam X (manufactured by Nippon NSC Ltd.), polymers described in Japanese Patent Application No. 2006-183144, and the like.

Polysaccharide-based hair setting resins:

Gum arabic, glucan, succinoglucan, carrageenan, karaya gum, tragacanth gum, guar gum, locust bean gum, galactomannam gum, xanthane gum, starch, carob gum, quince seed (marmelo), casein, dextrin, gelatin, sodium pectate, sodium alginate, methyl cellulose, ethyl cellulose, carboxymethyl cellulose, hydroxyethyl cellulose, hydroxypropyl cellulose, crystalline cellulose, O-[2-hydroxy-3-(trimethylammonio)propyl]hydroxyethyl cellulose chloride, O-[2-hydroxy-3-(trimethylammonio)propyl]guar gum chloride, O-[2-hydroxy-3-(trimethylammonio)propyl]locust bean gum chloride, hydroxypropyltrimonium starch chloride, and the like.

Although the hair setting resin incorporated in the hair-styling cosmetic of the present invention is not specifically limited and one kind or two kinds or more of the hair setting resins as listed above can be suitably selected and used, an acrylic-based, vinyl-based, amphoteric, cationic, nonionic or urethane-based hair setting resin is specifically preferable.

The incorporation amount of the hair setting resin in the hair-styling cosmetic of the present invention is generally from 0.1 to 30% by mass, preferably from 0.5 to 20% by mass, and more preferably from 1 to 15% by mass. When the amount is less than 0.1% by mass, humidity resistance may not be sufficient, and when the hair setting resin is incorporated by going beyond 30% by mass, hair may become stiff.

It is preferable that the hair-styling cosmetic of the present invention comprises water and an alcohol in addition to the above-mentioned adhesive resin and hair setting resin.

As the alcohol in the hair-styling cosmetic of the present invention, one kind or two kinds or more selected from alcohols that are generally used in cosmetics such as ethanol can be suitably selected and used. The incorporation amounts of the water and alcohol are not specifically limited, and are suitably adjusted within a range in which the above-mentioned predetermined adhesiveness and viscosity are given.

It is preferable that the hair-styling cosmetic of the present invention comprises an ultraviolet absorber in addition to the above-mentioned essential components.

The ultraviolet absorber used in the present invention is not specifically limited, and one kind or two kinds or more selected from those conventionally used in cosmetic and the like can be used.

Specific examples of the ultraviolet absorber may include benzoic acid-based ultraviolet absorbers (for example, paraaminobenzoic acid (hereinafter abbreviated as PABA), PABA monoglycerin ester, N,N-dipropoxyPABA ethyl ester, N,N-diethoxyPABA ethyl ester, N,N-dimethylPABA butyl ester, N,N-dimethylPABA ethyl ester and the like); anthranilic acid-based ultraviolet absorbers (for example, homomentyl-N-acetylanthranilate and the like); salicylic acid-based ultraviolet absorbers (for example, amyl salicylate, mentyl salicylate, homomentyl salicylate, octyl salicylate, phenyl salicylate, benzyl salicylate, p-isopropanolphenyl salicylate and the like); cinnamic acid-based ultraviolet absorbers (for example, octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate(2-ethylhexyl-p-methoxycinnamate), 2-ethoxyethyl-p-methoxycinnamate, cyclohexyl-p-methoxycinnamate, ethyl-$\alpha$-cyano-$\beta$-phenylcinnamate, 2-ethylhexyl-$\alpha$-cyano-$\beta$-phenylcinnamate, glyceryl mono-2-ethylhexanoyl-diparamethoxycinnamate and the like); benzophenone-based ultraviolet absorbers (for example, 2,4-dihydroxybenzophenone, 2,2'-dihydroxy-4-methoxybenzophenone, 2,2'-dihydroxy 4,4'-dimethoxybenzophenone, 2,2',4,4'-tetrahydroxybenzophenone, 2-hydroxy-4-methoxybenzophenone, 2 -hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone and the like); 3-(4'-methylbenzylidene)-d,l-camphor, 3-benzyliden.e-d,l-camphor; 2-phenyl-5-methylbenzoxazole; 2,2-hydroxy-5-methylphenylbenzotriazole;2-(2'-hydroxy-5'-t-octylphenyl)benzotriazole; 2-(2'-hydroxy-5'-methylphenylbenzotriazole;dibenzalazine; dianisoylmethane; 4-methoxy-4'-t-butyldibenzoylmethane;5-(3,3-dimethyl-2-norb ornilidene) -3-pentan-2-one; triazine derivatives (for example, bisethylhexyloxyphenolmethoxyphenyltriazine, methylenebisbenzotriazolyltetramethylbutylphenyltriazine, ethylhexyltriazone), and the like.

Since the hair-styling cosmetic of the present invention is a water-based one, when an ultraviolet absorber as mentioned above is incorporated, a water-soluble absorber can be incorporated as it is. Examples of the water-soluble ultraviolet absorber may include 2-phenylbenzimidazole-5-sulfonic acid such as "Neo Heliopan Hydro" (manufactured by Symrise) and "Eusolex232" (manufactured by Merck), and monosodium 2-hydroxy-4-methoxybenzophenone-5-sulfonate (benzophenone-5) such as "ASL-24S".

On the other hand, when an oil-soluble ultraviolet absorber (many of the above-mentioned specific examples are oil-soluble), it is preferable to use a solubilizing agent in combination except the case that the hair styling comprises a high content of ethanol and thus the oil-soluble ultraviolet absorber can be incorporated as it is.

As the solubilizing agent, a surfactant having an HLB of 10 or more is preferable. Examples may include polyoxyethylene cetyl ethers (Emalex 107, Emalex 110, Emalex 112, Emalex 115 , Emalex 117 and Emalex 120 (manufactured by Nihon Emulsion Co. , Ltd.)), polyoxyethylene oleyls (Emalex 508, Emalex 510, Emalex 512, Emalex 515 and Emalex 520 (manufactured by Nihon Emulsion Co. , Ltd.), polyoxyethylene octyl dodecyl ethers (for example, Emalex OD-16, Emalex Old-20 and Emalex OD-25 (manufactured by Nihon Emulsion Co., Ltd.), Nonion IP-220 (manufactured by NOF Corporation)), polyoxyethylene-hydrogenated castor oils (for example, Nikkol HCO-40, Nikkol HCO-50 and Nikkol HCO-60 (manufactured by Nihonsurfactant Kogyo K.K.), Emalex HC-40, Emalex HC-50 and Emalex HC-60 (manufactured by Nihon Emulsion Co., Ltd.), Emanon CH-40 and Emanon CH-60 (manufactured by Kao Corporation)), polyoxyethylenepolyoxypropylenedecyltetradecyl ethers (for example, S-Safe 1020, S-Safe 1324, S-Safe 1520, S-Safe 1525 (manufactured by NOF Corporation)), polyoxyethylenesorbitane aliphatic ester-based activators (for example, Emasol 120 series (manufactured by Kao Corporation), Rheodol TW series (manufactured by Kao Corporation) and Sorfon T series (manufactured by Toho Chemical Industry Co., Ltd.)), polyoxyethylene glyceryl laurates (for example, Unigly ML-220 and Unigly ML-230 (manufactured by NOF Corporation)), polyoxyethylene glyceryl oleates (for example, Unigly MO-220 and Unigly MO-230 (manufactured by NOF Corporation), polyoxyethylene phytosterols (for example, Nikkol BPS-20, Nikkol BPS-25 and Nikkol BPS-30 (manufactured by Nikko Chemicals Co., Ltd.), Bellpol DC-30 (manufactured by Nippon Fine Chemical), and SUNBRIGHT CS-010, SUNBRIGHT CS-020 and SUNBRIGHT CS-050 (manufactured by NOF Corporation)), and the like.

The hair-styling cosmetic of the present invention can be provided in various embodiments such as a hair liquid, a hair foam, a hair mousse, a hair spray, a hair mist, a hair gel and a hair wax, and is specifically suitable for a form for using by atomizing into a form of mist, since it is a cosmetic that comprises predetermined amount(s) of one kind or two kinds or more selected from sugar alcohols, polyalkylene glycols and derivatives thereof and has limited adhesiveness and viscosity. By using in a form of mist, the solvent volatilizes quickly, and adhesiveness and hair-styling effect can be exerted quickly.

The hair-styling cosmetic of the present invention may incorporate, depending on the form thereof, for example, other components that have been conventionally used in hair-styling cosmetics, to the extent that the effect of the invention is not deteriorated.

EXAMPLES

Hereinafter the present invention will be explained in more detail with referring to specific examples, but the present invention is not construed to be limited to the following Examples. Furthermore, the incorporation amounts in the following Examples and the like are represented by % by mass unless otherwise specified.

(Examples and Comparative Examples)

The compositions of the following Tables 1 to 4 were prepared, and the adhesiveness of each composition was measured by a ball tack test. In the tables, the result of the ball tack test represented by "-" shows that adhesiveness was so low that the ball of No. 1 did not stop.

Furthermore, samples were prepared by using the resins of the above-mentioned Preparation Examples and Comparative Preparation Examples, and the arrangement effect, fixing effect, hair-restyling effect and humidity resistance when the samples were used were evaluated.

The evaluation methods and evaluation criteria for the respective properties are as follows.

1. Arrangement effect

0.5 g of the sample was applied to a bundle of black virgin hair (length: 20 cm, mass: 2 g) and dried at an ordinary temperature, and the obtained hair bundle was evaluated for easiness of arrangement by a sensory test by specialized panellists.

<Criteria for evaluation points>

5 points: Arrangement is considerably easy
4 points: Arrangement is slightly easy
3 points: Normal
2 points: Arrangement is slightly difficult
1 point: Arrangement is difficult

<Evaluation criteria>

⊙: The total points are 40 points or more
○: The total points are 30 points or more and less than 40 points
△: The total points are 20 points or more and less than 30 points
✕: The total points are less than 20 points

2. Fixing effect

0.4 g of the sample was applied to black virgin hair (length: 15 cm, weight: 1 g) and spreaded by using a comb, the shape of the hair was arranged so that the hair was relaxed, and 5 strands were prepared for one sample. The strand was dried for 1 hour at 50˚C and hanged on a scaled board, and the flexure length (b) of the strand was measured in a constant temperature and humidity chamber at a temperature of 30˚C and a humidity of 90% RH. Using the flexure length (a) of the strand that had been measured in advance before applying the sample, fixing effect (keeping effect) was obtained according to the following equation. A numerical value closer to 100% represents higher fixing effect and more excellent humidity resistance.

$$\text{Hair style keeping effect (\%)} = \{(a-b)/a\} \times 100$$

<Evaluation criteria>

⊙: The value is 90% or more
○: The value is 70% or more and less than 90%
△: The value is 50% or more and less than 70%
✕: The value is less than 50%

3. Hair-restyling effect

0.5 g of the sample was applied to a bundle of black virgin hair (length: 20 cm, mass: 2 g) and dried at an ordinary temperature, and the obtained hair bundle was evaluated for easiness of hair styling when the hair was trimmed immediately after the application and trimmed again after 1 hour (hair-restyling effect) by a sensory test by specialized panelists.

<Criteria for evaluation points>

5 points: Hair-restyling effect is considerably high
4 points: Hair-restyling effect is slightly high
3 points: Normal
2 points: Hair-restyling effect is slightly low
1 point: Hair-restyling effect is not exhibited

<Evaluation criteria>

⊙: The total points are 40 points or more
○: The total points are 30 points or more and less than 40 points
△: The total points are 20 points or more and less than 30 points
×: The total points are less than 20 points

4. Humidity resistance

0.5 g of the sample (the hair cosmetic obtained in Example or Comparative Example) was applied to black virgin hair (length: 20 cm, weight: 2 g), and a curl was immediately made by using a curler having a curl diameter of 2 cm and dried at 50°C for 1 hour. The length of the curled hair strand was measured, and said length was defined as an initial value (L0).
Next, the dried hair bundle was hanged on a scaled board and put into a constant temperature and humidity chamber at a temperature of 30°C and a humidity of 90%RH for 3 hours, and the length of the hair strand was measured and defined as strength after humidification (L2).
The length of the hair strand is the maximum diameter when the hair is in a curled state, whereas the length is the maximum length from the end part at the base side (for example, the length from the end part at the base side to the end part of the tip) when the curl is uncoiled partially or entirely.
The curl retention value was calculated according to the following equation, and the humidity resistance was evaluated according to the following evaluation criteria.

$$\text{Curl retention value (\%)} = \{(20-L2)/(20-L0)\} \times 100$$

A curl retention value closer to 100% represents a stronger curl keeping rate and more excellent humidity resistance (i.e., style keeping effect).

<Evaluation criteria>

⊙: The curl retention value is 90% or more.
○: The curl retention value is 70% or more and less than 90%
△: The curl retention value is 50% or more and less than 70%
×: The curl retention value is less than 50%

[Table 1]

|  | Example 1 | Example 2 | Example 3 | Example 4 |
|---|---|---|---|---|
| Ion exchanged water | Amount that makes the entirety 100 | | | |
| Ethanol | 5 | - | - | - |
| 95% Synthesized alcohol | - | 20 | 20 | 20 |
| Multitol | 10 | - | - | - |
| Sorbitol | - | 5 | - | 5 |

(continued)

|  | Example 1 | Example 2 | Example 3 | Example 4 |
|---|---|---|---|---|
| PEG-6 | 7 | - | 2.5 | 2.5 |
| PEG-8 | - | - | - | - |
| PEG-32 | 7 | - | 2.5 | 2.5 |
| (Alkyl acrylate/diacetoneacrylamide)copolymer AMP | 3 | - | - | - |
| PEG/PPG-55/28 dimethyl ether | 1 | - | - | - |
| Vinylpyrrolidone/VA copolymer | - | - | 5 | - |
| Citric acid | - | - | - | - |
| Phenoxyethanol | Suitable amount | - | - | - |
| Fragrance | Suitable amount | - | - | - |
| Ball tack test (inclined angle 10˚, 25˚C) | 7 | 1 | 1 | 1 |
|  |  |  |  |  |
| Fixing effect | ⊙ | ○ | ○ | ○ |
| Arrangement effect | ○ | ○ | ○ | ○ |
| Hair-restyling effect | ⊙ | ○ | ○ | ○ |
| Humidity resistance | ○ | ○ | ○ | ○ |

[Table 2]

|  | Example 5 | Example 6 | Example 7 | Example 8 | Example 9 |
|---|---|---|---|---|---|
| Ion exchanged water | Amount that makes the entirety 100 | | | | |
| Ethanol | - | - | - | - | - |
| 95% Synthesized alcohol | 20 | 20 | 20 | 20 | 20 |
| Multitol | - | - | - | - | - |
| Sorbitol | 10 | 5 | 5 | 5 | 5 |
| PEG-6 | 5 | 2.5 | 2.5 | 2.5 | 2.5 |
| PEG-8 | - | - | - | - | - |
| PEG-32 | 5 | 2.5 | 2.5 | 2.5 | 2.5 |
| (Alkyl acrylate/diacetoneacrylamide)copolymer AMP | - | 1 | 5 | - | - |
| PEG/PPG-55/28 dimethyl ether | - | - | - | - | - |
| Vinylpyrrolidone/VA copolymer | - | - | - | 1 | 5 |
| Citric acid | - | - | - | - | - |
| Phenoxyethanol | - | - | - | - | - |
| Fragrance | - | - | - | - | - |
| Ball tack test (Inclined angle 10˚, 25˚C) | 3 | 2 | 1 | 2 | 1 |
|  |  |  |  |  |  |
| Fixing effect | ○ | ⊙ | ⊙ | ⊙ | ⊙ |

(continued)

|  | Example 5 | Example 6 | Example 7 | Example 8 | Example 9 |
|---|---|---|---|---|---|
| Arrangement effect | ⊙ | ○ | ○ | ○ | ○ |
| Hair-restyling effect | ⊙ | ○ | ○ | ○ | ○ |
| Humidity resistance | ○ | ○ | ⊙ | ○ | ⊙ |

[Table 3]

| | Comparative Example 1 | Comparative Example 2 | Comparative Example 3 | Comparative Example 4 | Comparative Example 5 | Comparative Example 6 |
|---|---|---|---|---|---|---|
| Ion exchanged water | Amount that makes the entirety 100 | | | | | |
| 95% Synthesized alcohol | 20 | 20 | 20 | 20 | 20 | 20 |
| Sorbitol | - | - | - | - | 1 | - |
| PEG-6 | - | - | - | - | - | 0.5 |
| PEG-32 | - | - | - | - | - | 0.5 |
| (Alkyl acrylate/diacetoneacrylamide) copolymer AMP | 5 | - | - | - | - | - |
| Vinylpyrrolidone/VA copolymer | - | 5 | - | - | - | - |
| Polyquaternium-11 | - | - | 5 | - | - | - |
| (Methecryloyloxyethylcarboxybetaine/ alkyl methacrylate) copolymer | - | - | - | 5 | - | - |
| Ball tack test (Inclined angle 10˚, 25˚C) | - | - | - | - | - | - |
| | | | | | | |
| Fixing effect | ○ | ○ | ○ | ○ | × | × |
| Arrangement effect | × | × | × | × | Δ | Δ |
| Hair-restyling effect | × | × | × | × | Δ | × |
| Humidity resistance | ○ | Δ | ○ | ○ | × | × |

[Table 4]

| | Comparative Example 7 | Comparative Example 8 | Comparative Example 9 | Comparative Example 10 | Comparative Example 11 | Comparative Example 12 |
|---|---|---|---|---|---|---|
| Ion exchanged water | Amount that makes the entirety 100 | | | | | |
| 95% Synthesized alcohol | 20 | 20 | 20 | 20 | 20 | 20 |
| Sorbitol | 0.5 | 1 | 1 | 1 | 1 | 1 |
| PEG-6 | 0.25 | 0.5 | 0.5 | 0.5 | 0.5 | 0.5 |
| PEG-32 | 0.25 | 0.5 | 0.5 | 0.5 | 0.5 | 0.5 |
| (Alkyl acrylate/diacetoneacrylamide) copolymer AMP | - | - | 1 | 5 | - | - |
| Vinylpyrrolidone/VA copolymer | - | - | - | - | 1 | 5 |
| Polyquaternium-11 | - | - | - | - | - | - |
| (Methecryloyloxyethylcarboxybetaine/ alkyl methacrylate) copolymer | - | - | - | - | - | - |
| Ball tack test (Inclined angle 10˚, 25˚C) | - | - | - | - | - | - |
| | | | | | | |
| Fixing effect | × | Δ | ○ | ○ | ○ | ○ |
| Arrangement effect | Δ | Δ | Δ | Δ | Δ | Δ |
| Hair-restyling effect | Δ | Δ | Δ | Δ | Δ | Δ |
| Humidity resistance | × | Δ | Δ | ○ | Δ | ○ |

**Claims**

1. A hair-styling cosmetic, comprising:

   (a) one kind or two kinds or more selected from sugar alcohols and
   (b) one kind or two kinds or more of polyethylene glycol (s) with 5 to 30% by mass in total,

   wherein the cosmetic has such an adhesiveness as showing a ball number of from 1 to 30 in an inclined ball tack test (inclination angle: 10˚, 25˚C, humidity: 50%) after forming a coating having a thickness of 0.1 mm by using the cosmetic and drying at 25˚C and a humidity of 50%,
   wherein the cosmetic has a viscosity of 100 mPa·s or less (25˚C, a B-type viscometer), and
   wherein the cosmetic is used by atomizing into a form of mist.

2. The hair-styling cosmetic according to claim 1, further comprising:

   (c) 0.1 to 10% by mass of a hair setting resin.

3. The hair-styling cosmetic according to claim 1 or 2,
   wherein the sugar alcohol (a) is one kind or two kinds or more selected from maltitol, sorbitol, ribitol, mannitol, arabitol, galactitol, xylitol and erythritol.

4. The hair-styling cosmetic according to any one of claims 1 to 3,
   wherein the polyethylene glycol (b) is one kind or two kinds or more selected from polyethylene glycols each having an average molecular weight of from 200 to 20,000.

5. The hair-styling cosmetic according to any one of claims 1 to 4,
   wherein the polyethylene glycol (b) is one kind or two kinds or more selected from the group consisting of PEG200, PEG300, PEG400, PEG600, PEG1000, PEG1540, PEG2000, PEG4000, PEG6000, PEG11000 and PEG20000.

6. The hair-styling cosmetic according to any one of claims 2 to 5,
   wherein the hair setting resin (c) is one kind or two kinds or more selected from acrylic-based, vinyl-based, amphoteric, cationic, nonionic and urethane-based hair setting resins.

[Figure 1]

Expressed in mm unit

<div align="center">

**INTERNATIONAL SEARCH REPORT**

</div>

| | International application No. |
|---|---|
| | PCT/JP2010/064048 |

A. CLASSIFICATION OF SUBJECT MATTER
*A61K8/34*(2006.01)i, *A61K8/02*(2006.01)i, *A61K8/60*(2006.01)i, *A61K8/72*
(2006.01)i, *A61Q5/06*(2006.01)i

According to International Patent Classification (IPC) or to both national classification and IPC

B. FIELDS SEARCHED

Minimum documentation searched (classification system followed by classification symbols)
A61K8/34, A61K8/02, A61K8/60, A61K8/72, A61Q5/06

Documentation searched other than minimum documentation to the extent that such documents are included in the fields searched
Jitsuyo Shinan Koho          1922-1996     Jitsuyo Shinan Toroku Koho      1996-2010
Kokai Jitsuyo Shinan Koho    1971-2010     Toroku Jitsuyo Shinan Koho      1994-2010

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2004-155724 A  (Mandom Corp.),<br>03 June 2004 (03.06.2004),<br>claims 1, 4, 5; paragraph [0017]; example 5<br>(Family: none) | 1-6 |
| Y | JP 2003-26545 A  (Kanebo, Ltd.),<br>29 January 2003 (29.01.2003),<br>claims 1 to 3; paragraphs [0001], [0017]<br>(Family: none) | 1-6 |
| A | JP 2005-281683 A  (Dai-Ichi Kogyo Seiyaku Co.,<br>Ltd.),<br>13 October 2005 (13.10.2005),<br>entire text<br>(Family: none) | 1-6 |

☒ Further documents are listed in the continuation of Box C.          ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 September, 2010 (24.09.10) | 05 October, 2010 (05.10.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/064048

C (Continuation).　DOCUMENTS CONSIDERED TO BE RELEVANT

| Category* | Citation of document, with indication, where appropriate, of the relevant passages | Relevant to claim No. |
|---|---|---|
| A | JP 2004-161764 A　(Wella AG.),<br>10 June 2004 (10.06.2004),<br>entire text<br>& US 2004/0115152 A1　　& EP 1302193 A2 | 1-6 |
| A | JP 2002-502908 A　(Union Carbide Chemicals &<br>Plastics Technology Corp.),<br>29 January 2002 (29.01.2002),<br>entire text<br>& US 6322811 B1　　　　& EP 1045885 A1<br>& WO 1999/040156 A1　　& CN 1296516 A | 1-6 |

Form PCT/ISA/210 (continuation of second sheet) (July 2009)

| **INTERNATIONAL SEARCH REPORT** | International application No. |
|---|---|
| | PCT/JP2010/064048 |

Claim 1 involves any hair-dressing cosmetics containing sugar alcohol(s) and polyethylene glycol(s) being "a cosmetic containing (a) one or more kinds of sugar alcohols and (b) one or more kinds of polyethylene glycols, in a total amount of 5 to 30 wt%", and having properties of showing a ball number of 1 to 30 "in the inclined ball tack test (inclination angle $10°$, $25°C$, humidity 50%) after forming a film of 0.1 mm in thickness using the cosmetic and drying the same for 1 day at $25°C$ and humidity 50%". However, cosmetics comprising specific sugar alcohols and polyethylene glycols at specific ratio as described in the description are exclusively disclosed in the meaning within PCT Article 5 and, therefore, the above claim is not supported in the meaning within PCT Article 6.

Thus, the search was made on the scope that is supported by the description, i.e., the cosmetics using the specific sugar alcohols and polyethylene glycols at specific ratio as described in Examples.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007217314 A
- JP H11100312 A
- JP H3261713 A
- JP 2002167317 A
- JP 2004505902 A
- JP 2006183144 A